(19) Europäisches Patentamt
European Patent Office
Office européen des brevets

(11) Veröffentlichungsnummer: **0 310 917**
**A2**

# (12) EUROPÄISCHE PATENTANMELDUNG

(21) Anmeldenummer: 88115877.8

(51) Int. Cl.4: **C07D 215/56 , A61K 31/47**

(22) Anmeldetag: 27.09.88

(30) Priorität: 09.10.87 DE 3734161
02.04.88 DE 3811341

(43) Veröffentlichungstag der Anmeldung:
12.04.89 Patentblatt 89/15

(84) Benannte Vertragsstaaten:
AT BE CH DE ES FR GB IT LI NL SE

(71) Anmelder: BAYER AG
Konzernverwaltung RP Patentabteilung
D-5090 Leverkusen 1 Bayerwerk(DE)

(72) Erfinder: Schriewer, Michael, Dr.
Am Thelen Siefen 1a
D-5068 Odenthal(DE)
Erfinder: Grohe, Klaus, Dr.
Am Wasserturm 10
D-5068 Odenthal(DE)
Erfinder: Petersen, Uwe, Dr.
Auf dem Forst 4
D-5090 Leverkusen 1(DE)

## (54) In 7-Stellung C-verknüpfte Chinolon- und 1,8-Naphthyridin-4-on-carbonsäuren , ein Verfahren zu ihrer Herstellung und ihre Verwendung als Zwischenprodukte für antibakterielle Chinolon- und 1,8-Naphthyridin-4-on-carbonsäuren.

(57) Die Erfindung betrifft neue in 7-Stellung Kohlenstoffsubstituierte 4-Chinolon-3-carbonsäurederivate und Naphthyridin-4-on-carbonsäurederivate der Formel I

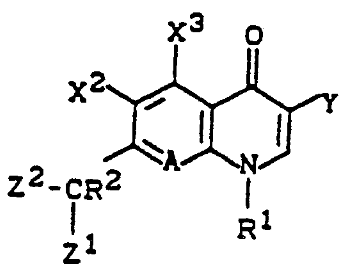

I

in der Y, $X^2$, $X^3$, A, $R^1$, $R^2$, $Z^1$ und $Z^2$ die in der Beschreibung angegebene Bedeutung haben, ein Verfahren zu ihrer Herstellung und ihre Verwendung als Zwischenprodukte für antibakterielle Chinolon- und 1,8-Naphthyridin-4-on-carbonsäuren.

EP 0 310 917 A2

## In 7-Stellung C-verknüpfte Chinolon- und 1,8-Naphthyridin-4-on-carbonsäuren, ein Verfahren zu ihrer Herstellung und ihre Verwendung als Zwischenprodukte für antibakterielle Chinolon- und 1,8-Naphthyridin-4-on-carbonsäuren

Die Erfindung betrifft neue in 7-Stellung Kohlenstoffsubstituierte 4-Chinolon-3-carbonsäurederivate und Naphthyridin-4-on-carbonsäurederivate, ein Verfahren zu ihrer Herstellung sowie deren Verwendung als Zwischenprodukte zur Herstellung antibakteriell hochwirksamer Chinoloncarbonsäuren und 1,8-Naphthyridin-4-on-carbonsäuren.

Beispielhaft sei auf die Verwendung als Zwischenprodukte für die Darstellung antibakteriell wirksamer Alkylchinoloncarbonsäuren hingewiesen, die in der Europäischen Patentanmeldung 0 181 588 beschrieben werden.

Nach dem erfindungsgemäßen Verfahren sind solche Verbindungen wesentlich leichter als bisher zugänglich. Dies betrifft insbesondere auch Derivate mit substituierten Alkylresten.

Gegenstand der Erfindung sind Verbindungen der Formel I sowie ein Verfahren zur Herstellung von Verbindungen der Formel I

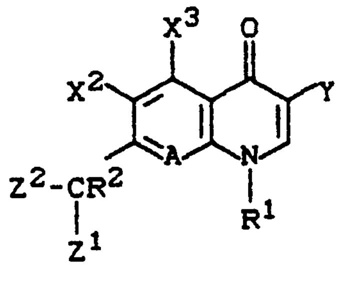

I

in der

Y eine Carboxylgruppe, eine Nitrilgruppe, eine Estergruppe $-COOR^3$ oder eine Säureamidgruppe $-CONR^4R^5$ darstellt, wobei $R^3$ für $C_1$-$C_4$-Alkyl steht, $R^4$ und $R^5$ für Wasserstoff oder $C_1$-$C_4$-Alkyl stehen, $R^5$ außerdem gegebenenfalls substituiertes Phenyl sein kann,

$X^2$ für Wasserstoff, Nitro, Alkyl mit 1 bis 3 Kohlenstoffatomen und Halogen insbesondere Chlor, Brom oder Fluor steht

$X^3$ Wasserstoff, Halogen insbesondere Chlor, Brom oder Fluor oder Methyl sein kann,

A für Stickstoff oder einen Rest $C$-$X^4$ steht, wobei $X^4$ Wasserstoff, Halogen, insbesondere Chlor, Brom oder Fluor, Nitro, Cyano oder Methyl sein kann

$R^1$ Alkyl mit 1-4 Kohlenstoffatomen, Vinyl, Halogenalkyl, Hydroxyalkyl, Cycloalkyl mit 1-6 Kohlenstoffatomen oder gegebenenfalls substituiertes Phenyl sein kann

$R^2$ für Wasserstoff, gegebenenfalls durch Halogen, Alkoxy mit 1-2 Kohlenstoffatomen oder Nitro substituiertes Alkyl mit 1-3 Kohlenstoffatomen, Phenyl sowie für einen Rest $NH\underset{\underset{O}{\|}}{C}R^6$

steht, wobei $R^6$ Wasserstoff, Alkyl mit 1-3 Kohlenstoffatomen sowie Phenyl bedeutet.

$Z^1$ und $Z^2$ gleich oder verschieden sein können und für Wasserstoff, Alkyl mit 1-3 Kohlenstoffatomen, gegebenenfalls substituiertes Phenyl, Pyridyl, Alkoxycarbonyl mit 1-4 Kohlenstoffatomen im Alkoholteil, Cyano, Isocyano, Nitro, Formyl, ferner für Reste $COR^7$, $SOR^8$, $SO_2R^9$, $SO_2OR^{10}$ und $SO_2NR^{11}R^{12}$ stehen können der Maßgabe, daß $Z^1$ und $Z^2$ nicht gleichzeitig Wasserstoff oder Alkyl sind. Außerdem können $Z^1$ und $Z^2$ mit dem Kohlenstoffatom, mit dem sie verknüpft sind, einen 5-oder 6-gliederigen Ring bilden, der als Ringglieder die Gruppen

$-O-$, $-\underset{\underset{O}{\|}}{C}-$, $-\underset{\underset{O}{\|}}{S}-$, $-SO_2-$, $>NR^{13}$ enthalten kann und an den

Kohlenstoffatomen ein- oder zweifach durch $C_1$-$C_3$-Alkyl, Phenyl, Halogen, Nitro, Cyano oder Alkoxycarbonyl mit 1-3 Kohlenstoffatomen im Alkoholteil substituiert ist, wobei

$R^7$, $R^8$, $R^9$ und $R^{10}$ für Alkyl mit 1-3 Kohlenstoffatomen oder gegebenenfalls substituiertes Phenyl stehen und

$R^{11}$ und $R^{12}$ gleich oder verslchieden sein können und für Wasserstoff, Alkyl mit 1-3 Kohlenstoffatomen oder gegebenenfalls substituiertes Phenyl stehen und

$R^{13}$ für $C_1$-$C_3$-Alkyl oder Phenyl steht.

Sie eignen sich als Zwischenprodukte für antibakteriell wirksame Chinoloncarbonsäuren und Naphthyridoncarbonsäuren.

Besonders bevorzugt sind Verbindungen der Formel I in der

Y Cyano oder eine Carbonestergruppe -$COOR^3$ darstellt, wobei $R^3$ für $C_1$-$C_4$-Alkyl steht

$X^2$ und $X^3$ für Wasserstoff und Halogen insbesondere Chlor oder Fluor stehen

A für Stickstoff oder einen Rest C-$X^4$ steht, wobei $X^4$ Wasserstoff, Halogen, insbesondere Chlor oder Fluor, Nitro oder Cyano sein kann

$R^1$ Ethyl, Vinyl, Cyclopropyl oder gegebenenfalls substituiertes Phenyl bedeutet

$R^2$ für Wasserstoff oder $C_1$-$C_3$-Alkyl steht,

$Z^1$ und $Z^2$ gleich oder verschieden sein können und für gegebenenfalls substituiertes Phenyl, Pyridyl, Alkoxycarbonyl mit 1-4 Kohlenstoffatome im Alkoholteil, Cyano, Isocyano, Nitro, Formyl oder den Rest $COR^7$ stehen können, wobei

$R^7$ für Alkyl mit 1-3 Kohlenstoffatomen steht.

Es wurde gefunden, daß man die erfindungsgemäßen Verbindungen der Formel I erhält, wenn man Verbindungen der Formel II

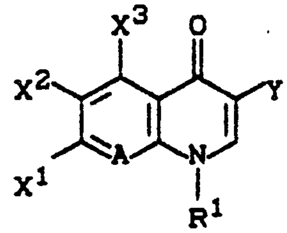

II

in der Y, A, $X^2$ und $X^3$ die oben angegebene Bedeutung haben und $X^1$ für Halogen, bevorzugt für Chlor und Fluor steht, mit Verbindungen der Formel III

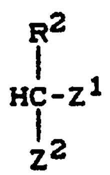

III

in der

$R^2$, $Z^1$ und $Z^2$ die oben angegebene Bedeutung haben in Gegenwart einer Base umsetzt.

Verwendet man beispielsweise 1-Cyclopropyl-6,7,8-Trifluor-1,4-dihydro-4-oxo-3-chinolin-carbonsäure-ethylester und Malonsäurediethylester als Ausgangsstoffe, so kann der Reaktionsablauf durch folgendes Formelschema wiedergegeben werden

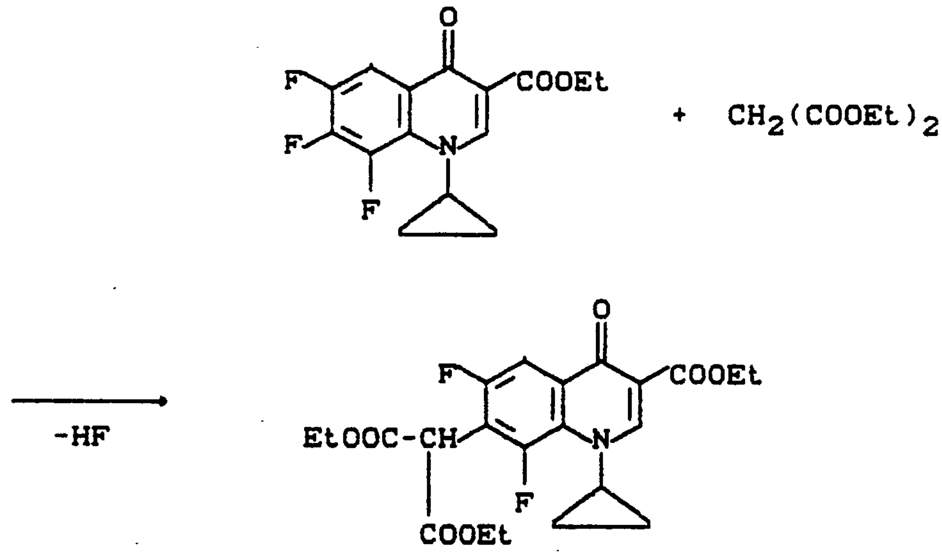

Die gemäß diesem Schema als Ausgangsstoffe benötigten Chinoloncarbonsäurederivate der Formel II sind bekannt oder können nach bekannten Methoden hergestellt werden. Als Beispiele seien genannt:
1-Cyclopropyl-6,7,8-trifluor-1,4-dihydro-4-oxo-3-chinolincarbonsäureethylester
1-Cyclopropyl-6,7-difluor-1,4-dihydro-4-oxo-3-chinolincarbonsäureethylester
7-Chlor-1-cyclopropyl-6-fluor-1,4-dihydro-4-oxo-3-chinolincarbonsäureethylester
6,8-Dichlor-1-cyclopropyl-7-fluor-1,4-dihydro-4-oxo-3-chinolincarbonsäureethylester
8-Chlor-1-cyclopropyl-6,7-difluor-1,4-dihydro-4-oxo-3-chinolincarbonsäureethylester
6-Chlor-1-cyclopropyl-7,8-difluor-1,4-dihydro-4-oxo-3-chinolincarbonsäureethylester
7-Chlor-1-cyclopropyl-6-fluor-1,4-dihydro-4-oxo-1,8-naphthyridin-3-carbonsäureethylester
6,7-Dichlor-1-cyclopropyl-1,4-dihydro-4-oxo-1,8-naphthyridin-3-carbonsäureethylester
1-Cyclopropyl-6,7,8-trifluor-1,4-dihydro-4-oxo-3-chinolincarbonsäureethylester
7-Chlor-6-fluor-1,4-dihydro-4-oxo-1-phenyl-1,8-naphthyridin-3-carbonsäureethylester
7-Chlor-6-fluor-1-(4-fluorphenyl)-1,4-dihydro-4-oxo-1,8-naphthyridin-3-carbonsäureethylester
7-Chlor-6-fluor-1-ethyl-1,4-dihydro-4-oxo-3-chinolincarbonsäuremethylester
7-Chlor-1-cyclopropyl-6-fluor-1,4-dihydro-4-oxo-3-chinolincarbonitril
7-Chlor-1-cyclopropyl-1,4-dihydro-4-oxo-3-chinolincarbonitril
7-Chlor-1-cyclopropyl-1,4-dihydro-6-nitro-4-oxo-3-chinolincarbonsäureethylester
6,7-Dichlor-1-cyclopropyl-1,4-dihydro-4-oxo-3-chinolincarbonsäureethylester
1-Cyclopropyl-7-chlor-6-fluor-1,4-dihydro-8-nitro-4-oxo-3-chinolincarbonsäureethylester
6,7-Difluor-1-ethyl-1,4-dihydro-4-oxo-3-chinolincarbonsäureethylester
7-Chlor-6-fluor-1-ethyl-1,4-dihydro-4-oxo-3-chinolincarbonsäuremethylester
6,7-Difluor-1-(2-fluorethyl)-1,4-dihydro-4-oxo-3-chinolincarbonsäureethylester
7-Chlor-6-fluor-1,4-dihydro-1-methoxy-4-oxo-3-chinolincarbonsäureethylester
6,7-Difluor-1,4-dihydro-4-oxo-1-phenyl-3-chinolincarbonsäureethylester
7-Chlor-6-fluor-1,4-dihydro-8-nitro-4-oxo-1-phenyl-3-chinolincarbonsäureethylester
7-Chlor-6-fluor-1-(4-fluorphenyl)-1,4-dihydro-8-nitro-4-oxo-3-chinolincarbonsäureethylester
6,7-Difluor-1-(4-fluorphenyl)-1,4-dihydro-8-methyl-4-oxo-3-chinolincarbonsäureethylester
6-Chlor-7-fluor-1-(4-fluorphenyl)-1,4-dihydro-4-oxo-3-chinolincarbonsäureethylester
6-Chlor-7-fluor-1-(2,4-difluorphenyl)-1,4-dihydro-4-oxo-3-chinolincarbonsäureethylester
6,7,8-Trifluor-1-(4-fluorphenyl)-1,4-dihydro-4-oxo-3-chinolincarbonsäureethylester
6,7,8-Trifluor-1-(2,4-difluorphenyl)-1,4-dihydro-4-oxo-3-chinolincarbonsäureethylester
6,7,8-Trifluor-1,4-dihydro-4-oxo-1-phenyl-3-chinolincarbonsäureethylester
7-Chlor-1-ethyl-6-fluor-1,4-dihydro-4-oxo-1,8-naphthyridin-3-carbonsäureethylester
6,7-Difluor-1,4-dihydro-4-oxo-1-vinyl-3-chinolincarbonsäureethylester.

Die als Ausgangsstoffe benötigten Verbindungen der Formel III sind bekannt. Als Beispiele seien genannt;
Malonsäurediethylester, Malonsäuredimethylester, Malondinitril, Cyanessigsäuremethylester, Cyanessigsäureethylester, Cyanessigsäure-t-butylester, Benzylcyanis, 4-Nitro-benzylcyanid, 2-Nitro-benzylcyanid, 2-Pyridylessigsäurenitril, 3-Pyrdidylessigsäurenitril, Acetessigsäureethylester, Benzylmethylsulfoxid, Benzylme-

thylsulfon, 2-Benzylpyridin, 4-Benzylpyridin, Isocyanessigsäureethylester, Benzylisocyant.

Die Umsetzung von II mit III wird vorzugsweise in einem Verdünnungsmittel wie Dimethylformamid, Dimethylsulfoxid, Hexamethylphosphorsäuretrisamid, Sulfolan, Dioxan, Tetrahydrofuran oder Pyridin vorgenommen. Ebenso könne Gemische dieser Verdünnungsmittel verwendet werden.

Als Basen können eingesetzt werden:
Natriumamid, Natriumhydrid, Kalium-t-butylat, n-Butyllithium, Phenyllithium.

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch beierhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol Carbonsäurederivat (II) 1 bis 5 Mol vorzugsweise 1 bis 3 Mol der Verbindung III ein. Auf ein Mol der Verbindung III setzt man ein Mol einer Base ein, wobei es vorteilhaft sein kann, einen Überschuß von 10 % einzusetzen.

Außer den in den Beispielen erwähnten Verbindungen seien folgende Derivate genannt: 7-(Cyano-ethoxycarbonyl-methyl)-1-cyclopropyl-6,8-difluor-1,4-dihydro-4-oxo-3-chinolincarbonitril,
7-(Cyano-methoxycarbonyl-methyl)-1-cyclopropyl-6,8-difluor-1,4-dihydro-4-oxo-3-chinolincarbonitril,
1-Cyclopropyl-7-(bis-ethoxycarbonyl-methyl)-6,8-difluor-1,4-dihydro-4-oxo-3-chinolincarbonitril,
7-(Cyano-phenyl-methyl)-1-cyclopropyl-6,8-difluor-1,4-dihydro-4-oxo-3-chinolincarbonitril,
1-Cyclopropyl-6,8-difluor-1,4-dihydro-7-(methylsulfonyl-phenyl-methyl)-4-oxo-3-chinolincarbonitril,
7-(3-Acetylacetonyl)-1-cyclopropyl-6,8-difluor-1,4-dihydro-4-oxo-3-chinolincarbonitril,
7-(Cyano-ethoxycarbonyl-methyl)-1-cyclopropyl-6,8-difluor-1,4-dihydro-4-oxo-3-chinolincarbonsäuremethylester,
7-(Cyano-methoxycarbonyl-methyl)-1-cyolopropyl-6,8-difluor-1,4-dihydro-4-oxo-3-chinolincarbonsäuremethylester,
1-Cyclopropyl-7-(bis-ethoxycarbonyl-methyl)-6,8-difluor-1,4-dihydro-4-oxo-3-chinolincarbonsäuremethylester,
7-(Cyano-phenyl-methyl)-1-cyclopropyl-6,8-difluor-1,4-dihydro-4-oxo-3-chinolincarbonsäuremethylester,
1-Cyclopropyl-6,8-difluor-1,4-dihydro-7-(methylsulfonyl-phenyl-methyl)-4-oxo-3-chinolincarbonsäuremethylester,
7-(3-Acetylacetonyl)-1-cyclopropyl-6,8-difluor-1,4-dihydro-4-oxo-3-chinolincarbonsäuremethylester,
6-Chlor-7-(cyano-ethoxycarbonyl-methyl-1-cyclopropyl-8-fluor-1,4-dihydro-4-oxo-3-chinolincarbonsäureethylester,
6-Chlor-7-(cyano-methoxycarbonyl-methyl)-1-cyclopropyl-8-fluor-1,4-dihydro-4-oxo-3-chinolincarbonsäureethylester,
6-Chlor-1-cyclopropyl-7-(bis-ethoxycarbonyl-methyl)-8-fluor-1,4-dihydro-4-oxo-3-chinolincarbonsäureethylester,
6-Chlor-7-(cyano-phenyl-methyl)-1-cyclopropyl-8-fluor-1,4-dihydro-4-oxo-3-chinolincarbonsäureethylester,
6-Chlor-1-cyclopropyl-8-fluor-1,4-dihydro-7-(methylsulfonyl-phenyl-methyl)-4-oxo-3-chinolincarbonsäureethylester,
7-(3-Acetylacetonyl)-6-chlor-1-cyclopropyl-8-fluor-1,4-dihydro-4-oxo-3-chinolincarbonsäureethylester,
8-Chlor-7-(cyano-ethoxycarbonyl-methyl)-1-cyclopropyl-6-fluor-1,4-dihydro-4-oxo-3-chinolincarbonsäureethylester,
8-Chlor-7-(cyano-methoxycarbonyl-methyl)-1-cyclopropyl-6-fluor-1,4-dihydro-4-oxo-3-chinolincarbonsäureethylester,
8-Chlor-1-cyclopropyl-7-(bis-ethoxycarbonyl-methyl)-6-fluor-1,4-dihydro-4-oxo-3-chinolincarbonsäureethylester,
8-Chlor-7-(cyano-phenyl-methyl)-1-cyclopropyl-6-fluor-1,4-dihydro-4-oxo-3-chinolincarbonsäureethylester,
8-Chlor-1-cyclopropyl-6-fluor-1,4-dihydro-7-(methylsulfonyl-phenyl-methyl-4-oxo-3-chnolincarbonsäureethylester,
7-(3-Acetylacetonyl)-8-chlor-1-cyclopropyl-6-fluor-1,4-dihydro-4-oxo-3-chinolincarbonsäureethylester,
7-(Cyano-methoxycarbonyl-methyl)-6,8-difluor-1,4-dihydro-4-oxo-1-phenyl-3-chinolincarbonsäureethylester,
7-(Bis-ethoxycarbonyl-methyl)-6,8-difluor-1,4-dihydro-4-oxo-1-phenyl-3-chinolincarbonsäureethylester,
7-(Cyano-phenyl-methyl)-6,8-difluor-1,4-dihydro-4-oxo-1-phenyl-3-chinolincarbonsäureethylester,
6,8-Difluor-1,4-dihydro-7-(methylsulfonyl-phenylmethyl)-4-oxo-1-phenyl-3-chinolincarbonsäureethylester,
7-(3-Acetylacetonyl)-6,8-difluor-1,4-dihydro-4-oxo-1-phenyl-3-chinolincarbonsäureethylester,
7-(Cyano-ethoxycarbonyl-methyl)-6,8-difluor-1,4-dihydro-4-oxo-1-vinyl-3-chinolincarbonsäureethylester,
7-(Cyano-methoxycarbonyl-methyl)-6,8-difluor-1,4-dihydro-4-oxo-1-vinyl-3-chinolincarbonsäureethylester,
7-(Bis-ethoxycarbonyl-methyl)-6,8-difluor-1,4-dihydro-4-oxo-1-vinyl-3-chinolincarbonsäureethylester,
7-(Cyano-phenyl-methyl)-6,8-difluor-1,4-dihydro-4-oxo-1-vinyl-3-chinolincarbonsäureethylester,

6,8-Difluor-1,4-dihydro-7-(methylsulfonyl-phenylmethyl)-4-oxo-1-vinyl-3-chinolincarbonsäureethylester,
7-(3-Acetylacetonyl)-6,8-difluor-1,4-dihydro-4-oxo-1-vinyl-3-chinolincarbonsäureethylester,
7-(Cyano-ethoxycarbonyl-methyl)-1-ethyl-6,8-difluor-1,4-dihydro-4-oxo-3-chinolincarbonsäureethylester,
7-(Cyano-methoxycarbonyl-methyl)-1-ethyl-6,8-difluor-1,4-dihydro-4-oxo-3-chinolincarbonsäureethylester,
7-(Bis-ethoxycarbonyl-methyl)-1-ethyl-6,8-difluor-1,4-dihydro-4-oxo-3-chinolincarbonsäureethylester,
7-(Cyano-phenyl-methyl)-1-ethyl-6,8-difluor-1,4-dihydro-4-oxo-3-chinolincarbonsäureethylester,
1-Ethyl-6,8-difluor-1,4-dihydro-7-(methylsulfonylphenyl-methyl)-4-oxo-3-chinolincarbonsäureethylester,
7-(3-Acetylacetonyl)-1-ethyl-6,8-difluor-1,4-dihydro-4-oxo-3-chinolincarbonsäureethylester,
7-(Cyano-ethoxycarbonyl-methyl)-1-cyclopropyl-6-fluor-1,4-dihydro-4-oxo-1,8-naphthyridin-3-chinolincarbonsäureethylester,
7-(Cyano-methoxycarbonyl-methyl)-2-cyclopropyl-6-fluor-1,4-dihydro-4-oxo-1,8-naphthyridin-3-carbonsäureethylester,
1-Cyclopropyl-7-(bis-ethoxycarbonyl-methyl)-6-fluor1,4-dihydro-4-oxo-1,8-naphthyridin-3-carbonsäureethylester,
7-(Cyano-phenyl-methyl)-1-cyclopropyl-6-fluor-1,4-dihydro-4-oxo-1,8-naphthyridin-3-carbonsäureethylester,
1-Cyclopropyl-6-fluor-1,4-dihydro-7-(methylsulfonylphenyl-methyl)-4-oxo-1,8-naphthyridin-3-carbonsäureethylester,
7-(3-Acetylacetonyl)-1-cyclopropyl-6-fluor-1,4-dihydro-4-oxo-1,8-napthyridin-3-carbonsäureethylester,
1-Cyclopropyl-6,8-difluor-1,4-dihydro-7-(2,2-dimethyl-1,3-dioxan-4,6-dion-5-yl)-4-oxo-3-chinolincarbonsäureethylester,
1-Cyclopropyl-6-fluor-1,4-dihydro-7-(2,2-dimethyl-1,3-dioxan-4,6-dion-5-yl)-4-oxo-3-chinolincarbonsäureethylester,
6-Chlor-1-cyclopropyl-8-fluor-1,4-dihydro-7-(2,2-dimethyl-1,3-dioxan-4,6-dion-5-yl)-4-oxo-3-chinolincarbonsäureethylester,
6,8-Difluor-1,4-dihydro-7-(2,2-dimethyl-1,3-dioxan-4,6-dion-5-yl)-4-oxo-1-phenyl-3-chinolincarbonsäureethylester,
1-Cyclopropyl-6-fluor-1,4-dihydro-7-(2,2-dimethyl-1,3-dioxan-4,6-dion-5-yl)-4-oxo-1,8-naphthyridin-3-carbonsäureethylester,
1-Cyclopropyl-6,8-difluor-1,4-dihydro-7-(1,3-dimethylhexahydropyrimidin-2,4,6-trion-5-yl)-4-oxo-3-chinolincarbonsäureethylester,
1-Cyclopropyl-6-fluor-1,4-dihydro-7-(1,3-dimethyl-hexahydropyrimidin-2,4,6-trion-5-yl)-4-oxo-3-chinolincarbonsäureethylester,
6-Chlor-1-cyclopropyl-6-fluor-1,4-dihydro-7-(1,3-dimethyl-hexahydropyrimidin-2,4,6-trion-5-yl)-4-oxo-3-chinolincarbonsäure,
6,8-Difluor-1,4-dihydro-7-(1,3-dimethyl-hexahydropyrimidin-2,4,6-trion-5-yl)-4-oxo-1-phenyl-3-chinolincarbonsäureethylester,
1-Cyclopropyl-6-fluor-1,4-dihydro-7-(1,3-dimethyl-hexahydropyrimidin-2,4,6-trion-5-yl)-4-oxo-1,8-naphthyridin-3-carbonsäureethylester,

Die folgenden Beispiele erläutern die Erfindung:

Beispiel 1

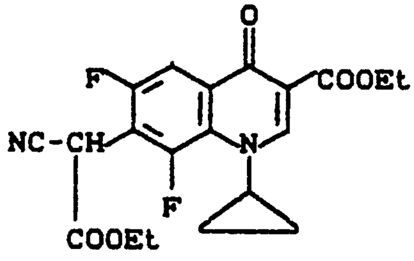

7-(Cyano-ethoxycarbonyl-methyl)-1-cyclopropyl-6,8-difluor-1,4-dihydro-4-oxo-3-chinolincarbonsäureethylester

86,2 g 1-Cyclopropyl-6,7-trifluor-1,4-dihydro-4-oxo-3-chinolincarbonsäureethylester und 63,3 g Cyanessigsäureethylester werden in 1000 ml Dioxan vorgelegt. 15,8 g NaH werden portionsweise zugegeben. Anschließend wird 4 Stunden am Rückfluß gekocht. Die Vollständigkeit der Reaktion wird mit Dünnschichtchromatographie überprüft. Nach Beendigung der Reaktion wird Wasser zugetropft. Danach stellt man mit verd. HCl sauer und extrahiert mit Methylenchlorid. Die organische Phase wird getrocknet und eingedampft. Den Rückstand kristallisiert man aus Isopropanol um.
Ausbeute: 86,5 g; Schmelzpunkt; 136-37°.

| $C_{20}H_{18}F_2N_2O_5$ | | | | |
|---|---|---|---|---|
| ber | C 59,4 | H 4,5 | N 7,0 | F 9,4 |
| gef | 59,2 | 4,5 | 7,0 | 9,5 |
| | 59,0 | 4,0 | 6,9 | 9,5 |

Beispiel 2

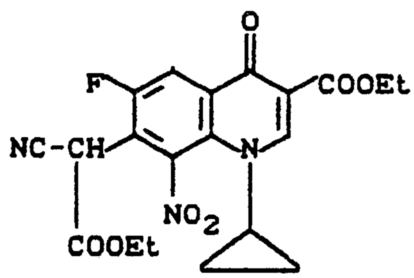

7-(Cyano-ethoxycarbonyl-methyl)-1-cyclopropyl-6-fluor-1,4-dihydro-8-nitro-4-oxo-3-chinolincarbonsäureethylester,

3,0 g 7-Chlor-1-cyclopropyl-6-fluor-1,4-dihydro-8-nitro-4-oxo-3-chinolincarbonsäureethylester und 1,92 g Cyanessigsäureethylester werden in 30 ml Dioxan vorgelegt. Bei Raumtemperatur werden 1,91 g Kalium tert.-butylat portionsweise zugegeben. Man läßt über Nacht rühren und erwärmt anschließend noch 4 Stunden auf 50°. Nach Abkühlen auf Raumtemperatur wird mit Eiswasser verdünnt und mit HCl sauer gestellt. Man extrahiert mit Methylenchlorid, trocknet die organische Phase und engt sie ein. Der Rückstand wird aus Isopropanol umkristallisiert.
Ausbeute: 2,0 g; Schmelzpunkt: 135-137°C.

| $C_{20}H_{18}F_2N_3O_7$ | | | |
|---|---|---|---|
| ber | C 55,7 | H 4,2 | N 9,7 |
| gef | 55,1 | 4,4 | 9,4 |
| | 55,3 | 4,6 | 9,2 |

Beispiel 3

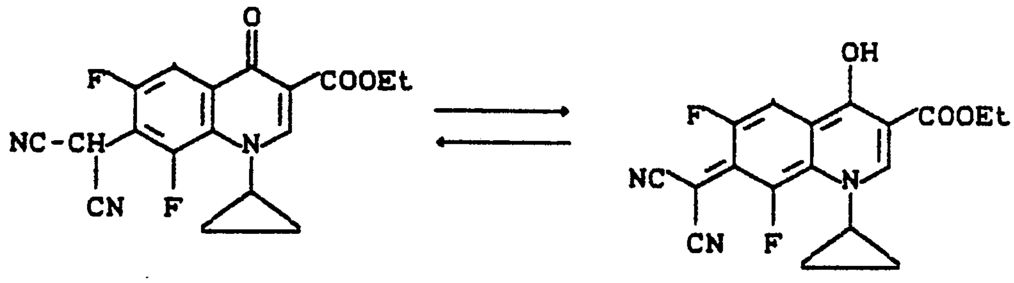

7-(Dicyano-methyl)-1-cyclopropyl-6,8-difluor-1,4-dihydro-4-oxo-3-chinolincarbonsäureethylester

2,7 g 1-Cyclopropyl-6,7,8-trifluor-1,4-dihydro-4-oxo-3-chinolincarbonsäureethylester und 1,1 g Malondinitril werden in 30 ml Dioxan vorgelegt. 1,91 g Kalium-tert.-butylat werden portionsweise zugefügt. Man erwärmt 4 Stunden auf 50°, kühlt aus Raumtemperatur, säuert mit HCl an und isoliert den roten Feststoff. Es wird aus Glykolmonomethylether umkristallisiert.
Ausbeute: 1,8 g; Schmelpunkt: 208-10° (Z)

| $C_{18}H_{13}F_2N_3O_3$ | | | | |
| --- | --- | --- | --- | --- |
| ber<br>gef | C 60,5<br>60,3 | H 3,6<br>4,1 | N 11,8<br>11,7 | F 10,6<br>10,5 |
| | 60,4 | 3,9 | 11,9 | 10,4 |

Nach NMR- und IR-Spektrum liegt die Verbindung praktisch ausschließlich als Enol vor.

Beispiel 4

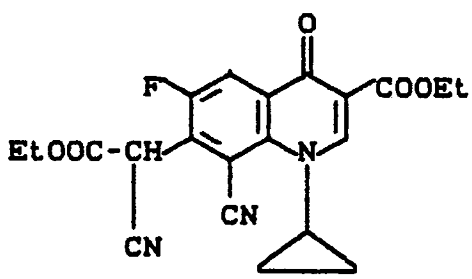

8-Cyano-7-(cyano-ethoxycarbonyl-methyl)-1-cyclopropyl-6-fluor-1,4-dihydro-4-oxo-3-chinolincarbonsäureethylester

2,8 g 7-Chlor-8-cyano-1-cyclopropyl-6-fluor-1,4-dihydro-4-oxo-3-chinolincarbonsäureethylester und 1,92 g Cyanessigsäureethylester werden in 30 ml Dioxan vorgelegt. Bei Raumtemperatur werden 1,91 g Kalium-tert.-butylat zudosiert. Man läßt über Nacht rühren und gibt dann Eiswasser zu. Es wird mit HCl sauer gestellt und mit Methylenchlorid extrahiert. Die organische Phase wird getrocknet und eingeengt. Der Rückstand wird noch einmal mit Wasser verrührt und der Feststoff isoliert.
Ausbeute: 2,0 g; Schmelzpunkt 95-115° (Z).

Umkristallisation aus Isopropanol liefert einen Feststoff mit dem Schmelzpunkt 158-59°.

Beispiel 5

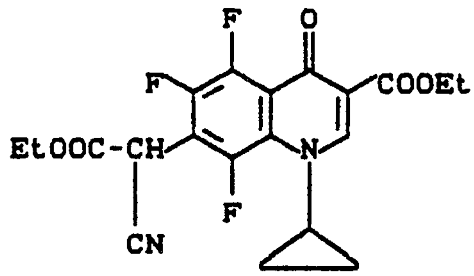

7-(Cyano-ethoxycarbonyl-methyl)-1-cyclopropyl-5,6,8-trifluor-1,4-dihydro-4-oxo-3-chinolincarbonsäureethylester

6,6 g 1-Cyclopropyl-5,6,7,8-tetrafluor-1,4-dihydro-4-oxo-3-chinolincarbonsäureethylester und 3,4 g Cyanessigsäureethylester werden in 100 ml Dioxan vorgelegt. Bei Raumtemperatur werden 1,16 g NaH portionsweise zugefügt. Man kocht 6 Stunden am Rückfluß und verdünnt dann mit Wasser. Nach Ansäuren mit HCl können 9,4 g der Titelverbindung isoliert werden.
Schmelzpunkt: 98-100°.

Die Verbindung kristallisiert mit einem Mol Dioxan (nachgewiesen durch NMR)

Beispiel 6

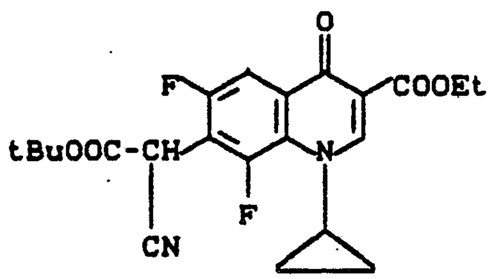

8-(t-Butoxycarbonyl-cyano-methyl)-1-cyclopropyl-6,8-difluor-1,4-dihydro-4-oxo-3-chinolincarbonsäureethylester

3,1 g Cyclopropyl-6,7,8-trifluor-1,4-dihydro-4-oxo-3-chinolincarbonsäureethylester und 2,1 g Cyanessigsäure-t-butylester werden in 30 ml Dioxan vorgelegt. 0,57 g Natriumhydrid werden portionsweise zugefügt. Man kocht 3 Stunden und gibt dann Eiswasser zu. Nach Ansäuern mit HCl wird mit $CH_2Cl_2$ extrahiert. Die organische Phase wird getrocknet und eingeengt. Der Rückstand wird aus Isopropanol umkristallisiert.
Ausbeute: 3,4 g; Schmelzpunkt: 143-45°.

Beispiel 7

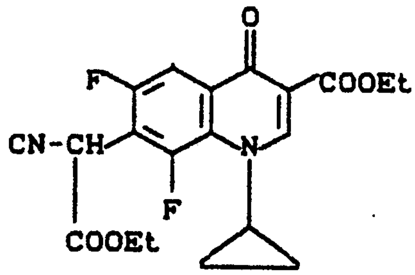

1-Cyclopropyl-7-(ethoxycarbonyl-isocyano-methyl)-6,8-difluor-1,4-dihydro-4-oxo-3-chinolincarbonsäureethylester

2,7 g 1-Cyclopropyl-6,7,8-trifluor-1,4-dihydro-4-oxo-3-chinolincarbonsäureethylester und 1,92 g Isocyanessigsäureethylester werden in 30 ml Dioxan vorgelegt. Man fügt 1,91 g Kalium-tert.-butylat zu und kocht 3 Stunden am Rückfluß. Anschließend werden noch einmal 0,39 g Kalium-tert.-butylat zugegeben und man kocht noch einmal 2 Stunden. Danach wird mit Wasser verdünnt, mit HCl angesäuert und der Feststoff isoliert. Nach Trennung an Kieselgel (Laufmittel $CH_2Cl_2$/MeOH 95/5) werden 1,0 g Titelverbindung erhalten. Schmelzpunkt: 68-71° (Z). IR 2149 $cm^{-1}$ ($-\overset{\oplus}{N}\equiv\overset{\ominus}{C}$ )

Beispiel 8

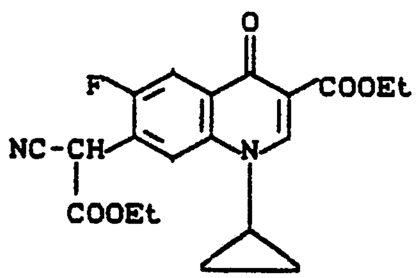

7-(Cyan-ethoxycarbonyl-methyl)-1-cyclopropyl-6-fluor-1,4-dihydro-4-oxo-3-chinolincarbonsäureethylester

8,8 g 1-Cyclopropyl-6,7-difluor-1,4-dihydro-4-oxo-3-chinolincarbonsäureethylester und 5,1 g Cyanessigsäureethylester werden in 120 ml Dioxan vorgelegt. 1,74 g NaH werden portionsweise zugegeben. Danach kocht man 3 Stunden, verdünnt mit Wasser und stellt mit HCL sauer. Es wird mit Methylenchlorid extrahiert. Die organische Phase wird getrocknet und eingeengt. Den Rückstand kristallisiert man aus Ethanol um. Ausbeute: 9,2 g; Schmelzpunkt 152-54°.

Beispiel 9

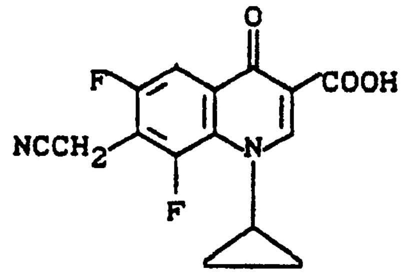

7-(Cyano-methyl)-cyclopropyl-6,8-difluor-1,4-dihydro-4-oxo-3-chinolincarbonsäure

100 g Verbindung aus Beispiel 1, 230 ml Essigsäure, 200 ml Wasser und 20 ml Schwefelsäure werden 5 Stunden gekocht. Nach Abkühlung aus Raumtemperatur wird Wasser zugefügt. Der ausgefallene Feststoff wird isoliert, mit Wasser gewaschen und getrocknet.
Ausbeute: 71,5 g; Schmelzpunkt 219-21°.

Beispiel 10

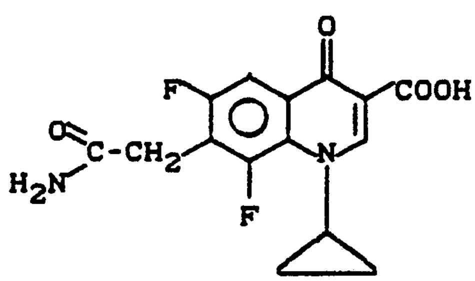

7-(Carbamoyl-methyl)-1-cyclopropyl-6,8-difluor-1,4-dihydro-4-oxo-3-chinolincarbonsäure

65 g Verbindung aus Beispiel 9 werden in 600 ml konzentrierte HCl eingetragen. Man läßt bei Raumtemperatur rühren, bis durch Dünnschichtchromatographie kein Edukt mehr nachweisbar ist. Danach wird auf Eis gegossen und der Niederschlag isoliert. Man wäscht mit viel Wasser und trocknet im Vakuumtrockenschrank. Nach Umkristallisation aus einem Gemisch von Glykolmonomethyletheracetat und Dimethylformamid werden 46 g der Titelverbindung erhalten.
Schmelzpunkt: 280-2° (Z).

| $C_{15}H_{12}F_2N_2O_4$ | | | |
|---|---|---|---|
| ber<br>gef | C 55,9<br>56,2 | H 3,7<br>3,9 | N 8,7<br>8,6 |
| | 56,3 | 3,8 | 8,8 |

Beispiel 11

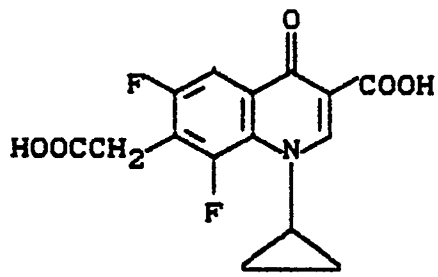

1-Cyclopropyl-6,8-difluor-1,4-dihydro-7-(hydroxycarbonyl-methyl)-4-oxo-3-chinolincarbonsäure

3,0 g Verbindung aus Beispiel 10 werden in 17 ml 30 %iger Schwefelsäure auf 90-100° C arwärmt. Bei dieser Temperatur werden 6 ml 2,5 molarer Natriumnitrit-Lösung zugetropft. Man rührt noch eine Stunde bei 100° und kühlt dann ab. Der ausgefallene Feststoff wird isoliert, gewaschen und getrocknet.
Ausbeute: 2,7 g; Schmelzpunkt 206-7° (Z).

| $C_{15}H_{11}F_2NO_5$ | | | |
|---|---|---|---|
| ber<br>gef | C 55,7<br>55,5 | H 3,4<br>3,7 | N 4,3<br>4,4 |
| | 55,2 | 3,4 | 4,3 |

Beispiel 12

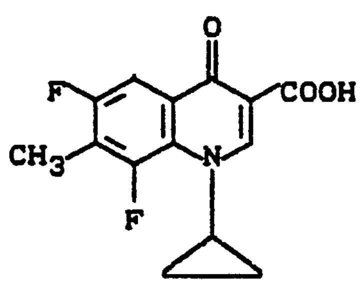

1-Cyclopropyl-6,8-difluor-1,4-dihydro-7-methyl-4-oxo-3-chinolincarbonsäure

0,5 g Verbindung aus Beispiel 11 werden in 5 ml Diphenylether eine Stunde auf 240° C erhitzt. Der nach Abdestillieren des Lösungsmittels verbleibende Rückstand besteht aus 0,3 g Rohprodukt vom Schmelzpunkt 208-10° C. Umkristallisation aus Ethanol ergibt einen Schmelzpunkt von 226-28°.

Diese Verbindung ist als antibakteriell sehr gut wirksam beschrieben. (EP O 181 588).

Beispiel 13

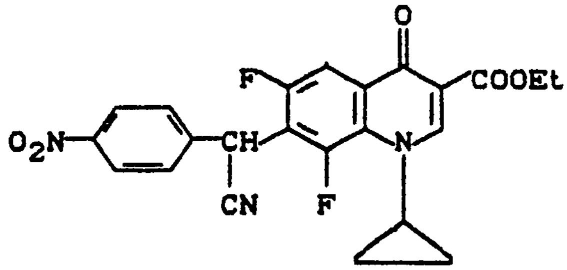

7-(Cyano-4-nitrophenyl-methyl)-1-cyclopropyl-6,8-difluor-1,4-dihydro-4-oxo-3-chinolincarbonsäureethylester

3,3 g 4-Nitro-benzylnitril werden in 24 ml DMSO vorgelegt. Anschließend gibt man 0,6 g Natriumhydrid zu. Nach Ende der Gasentwicklung werden 4,0 g 1-Cyclopropyl-6,7,8-trifluor-1,4-dihydro-4-oxo-3-chinolincarbonsäureethylester zugegeben. Das Reaktionsgemisch wird 6 Stunden auf 100° C erwärmt. Danach wird Wasser zugegeben und mit HCl sauer gestellt. Der Feststoff wird isoliert, gewaschen und getrocknet. Die Trennung an Kieselgel (Laufmittel Methylenchlorid/Methanol 96/4) liefert 4,8 g der Titelverbindung vom Schmelzpunkt 85-87° (Z).

Beispiel 14

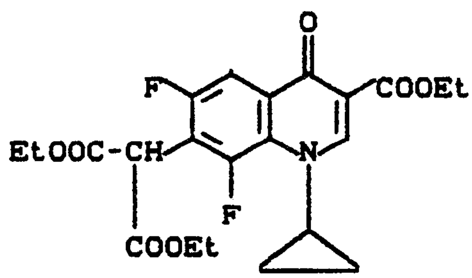

1-Cyclopropyl-7-(bis-ethoxycarbonyl-methyl)-6,8-difluor-1,4-dihydro-4-oxo-3-chinolincarbonsäureethylester

2,0 g Verbindung aus Beispiel 3 werden in 30 ml Ethanol vorgelegt. Anschließend wird 30 Minuten HCl-Gas eingeleitet. Man kocht 3 Stunden am Rückfluß, kühlt ab, gibt Wasser zu und extrahiert mit Methylenchlorid. Die organische Phase wird getrocknet und eingedampft. Der Rückstand wird aus Ethanol umkristallisiert.
Ausbeute: 1,2 g; Schmelzpunkt: 128-29°.

Beispiel 15

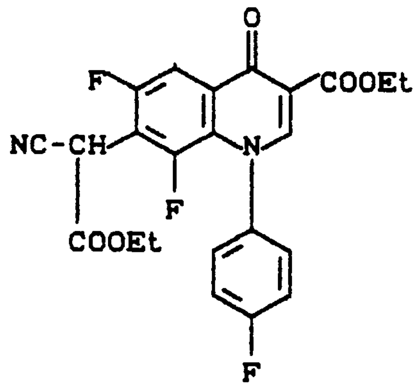

7-(Cyano-ethoxycarbonyl-methyl)-6,8-difluor-phenyl)-1,4-dihydro-4-oxo-3-chinolincarbonsäureethylester

0,12 g Natriumhydrid werden 15 ml Dioxan vorgelegt. Man gibt 0,51 g Cyanessigsäureethylester zu und läßt zunächst eine Stunde bei Raumtemperatur rühren. Danach werden portionsweise 1,1 g 6,7,8-Trifluor-1-(4-fluor-phenyl)-1,4-dihydro-4-oxo-3-chinolincarbonsäureethylester zugefügt. Man läßt 3 Stunden kochen, verdünnt mit Wasser und säuert an. Es wird mit methylenchlorid extrahiert. Die organische Phase wird getrocknet und eingeengt. Der Rückstand wird aus Ethanol umkristallisiert.
Ausbeute: 0,9 g; Schmelzpunkt: 172-73°.

## Ansprüche

1. Chinoloncarbonsäuren bzw. 1,8-Naphthyridin-4-on-carbonsäuren der Formel I

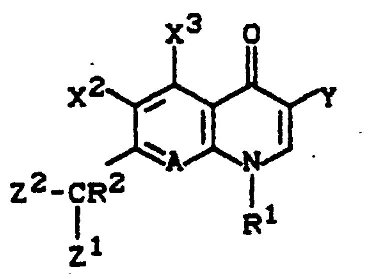

I

in der

Y eine Carboxylgruppe, ein Nitrilgruppe, eine Estergruppe $-COOR^3$ order ein Säureamidgruppe $-CONR^4R^5$ darstellt, wobei $R^3$ für $C_1$-$C_4$-Alkyl steht, $R^4$ und $R^5$ für Wasserstoff oder $C_1$-$C_4$-Alkyl stehen, $R^5$ außerdem gegebenenfalls substituiertes Phenyl sein kann,

$X^2$ für Wasserstoff, Nitro, Alkyl mit 1 bis 3 Kohlenstoffatomen und Halogen, insbesondere Chlor, Brom oder Fluor steht

$X^3$ Wasserstoff, Halogen, insbesondere Chlor, Brom oder Fluor oder Methyl sein kann,

A für Stickstoff oder einen Rest $C$-$X^4$ steht, wobei $X^4$ Wasserstoff, Halogen, insbesondere Chlor, Brom order Fluor, Nitro, Cyano oder Methyl sein kann

$R^1$ Alkyl mit 1-4 Kohlenstoffatomen, Vinyl, Halogenalkyl, Hydroxyalkyl, Cycloalkyl mit 1-6 Kohlenstoffatomen oder gegebenenfalls substituiertes Phenyl sein kann

$R^2$ für Wasserstoff, gegebenenfalls durch Halogen, Alkoxy mit 1-2 Kohlenstoffatomen oder Nitro substituiertes Alkyl mit 1-3 Kohlenstoffatomen, Phenyl
sowie für einen Rest $NH\,\underset{\overset{\|}{O}}{C}\,R^6$
steht, wobei

$R^6$ Wasserstoff, Alkyl mit 1-3 Kohlenstoffatomen sowie Phenyl bedeutet.

$Z^1$ und $Z^2$ gleich oder verschieden sein können und für Wasserstoff, Alkyl mit 1-3 Kohlenstoffatomen, gegebenenfalls substitiertes Phenyl, Pyridyl, Alkoxycarbonyl mit 1-4 Kohlenstoffatomen im Alkoholteil,

Cyano, Isocyano, Nitro, Formyl, ferner für Reste $COR^7$, $SOR^8$, $SO_2R^9$, $SO_2OR^{10}$ und $SO_2NR^{11}R^{12}$ stehen können mit der Maßgabe, daß $Z^1$ und $Z^2$ nicht gleichzeitig Wasserstoff und Alkyl sind. Außerdem können $Z^1$ und $Z^2$ mit dem Kohlenstoffatom, mit dem sie verknüpft sind, einen 5-oder 6-gliedrigen Ring bilden, der als Ringglieder die Gruppen

-O-, $-\overset{O}{\overset{\|}{C}}-$, $-\overset{O}{\overset{\|}{S}}-$, $-SO_2-$, $>NR^{13}$ enthalten kann und an den

Kohlenstoffatomen ein- oder zweifach durch $C_1$-$C_3$-Alkyl, Phenyl, Halogen, Nitro, Cyano oder Alkoxycarbonyl mit 1-3 Kohlenstoffatomen im Alkoholteil substituiert ist, wobei

$R^7$, $R^8$, $R^9$ und $R^{10}$ für Alkyl mit 1-3 Kohlenstoffatomen oder gegebenenfalls substituiertes Phenyl stehen und

$R^{11}$ und $R^{12}$ gleich oder verschieden sein können und für Wasserstoff, Alkyl mit 1-3 Kohlenstoffatomen oder gegebenenfalls substituiertes Phenyl stehen und

$R^{13}$ für $C_1$-$C_3$-Aklyl Phenyl steht,

sowie deren physiologisch verträgliche Salze, Ester und Prodrugs.

2. Verbindungen der Formel I gemäß Anspruch 1, in der

Y Cyano oder eine Carbonestergruppe $-COOR^3$ darstellt, wobei $R^3$ für $C_1$-$C_4$-Alkyl steht

$X^2$ und $X^3$ für Wasserstoff, Chlor oder Fluor stehen

A für Stickstoff oder einen Rest $C-X^4$ steht, wobei $X^4$ Wasserstoff, Chlor oder Fluor, Nitro oder Cyano sein kann

$R^1$ Ethyl, Vinyl, Cyclopropyl oder gegebenenfalls substituiertes Phenyl bedeutet

$R^2$ für Wasserstoff oder $C_1$-$C_3$-Alkyl steht,

$Z^1$ und $Z^2$ gleich oder verschieden sein können und für gegebenenfalls substituiertes Phenyl, Pyridyl, Alkoxycarbonyl wie 1-4 Kohlenstoffatome im Alkoholteil, Cyano, Isocyano, Nitro, Formyl oder den Rest $COR^7$ stehen können, wobei

$R^7$ für Alkyl mit 1-3 Kohlenstoffatomen steht.

3. Verfahren zur Herstellung von Chinoloncarbonsäuren bzw. 1,8-Naphthyridin-4-on-carbonsäuren der Formel I

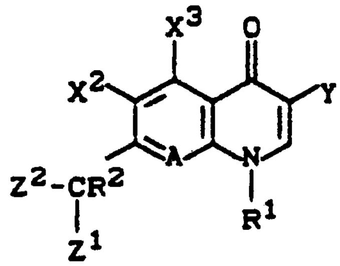

I

in der

Y eine Carboxylgruppe, eine Nitrilgruppe, eine Estergruppe $-COOR^3$ oder ein Säureamidgruppe $-CONR^4R^5$ darstellt, wobei $R^3$ für $C_1$-$C_4$-Alkyl steht, $R^4$ und $R^5$ für Wasserstoff oder $C_1$-$C_4$- Alkyl stehen, $R^5$ außerdem gegebenenfalls substituiert Phenyl sein kann,

$X^2$ für Wasserstoff, Nitro, Alkyl mit 1 bis 3 Kohlenstoffatomen und Halogen, insbesondere Chlor, Brom oder Fluor steht

$X^3$ Wasserstoff, Halogen, insbesondere Chlor, Brom oder Fluor oder Methyl sein kann,

A für Stickstoff oder einen Rest $C-X^4$ steht, wobei $X^4$ Wasserstoff, halogen insbesonder Chlor, Brom oder Fluor, Nitro, Cyano oder Methyl sein kann

$R^1$ Alkyl mit 1-4 Kohlenstoffatomen, Vinyl, Halogenalkyl, Hydroxyalkyl, Cycloalkyl mit 1-6 Kohlenstoffatomen oder gegebenenfalls substituiertes Phenyl sein kann

$R^2$ für Wasserstoff, gegebenenfalls durch Halogen, Alkoxy mit 1-2 Kohlenstoffatomen oder Nitro substituiertes Alkyl mit 1-3 Kohlenstoffatomen, Phenyl

sowie für einen Rest $NH\overset{O}{\overset{\|}{C}}R^6$

steht, wobei

$R^6$ Wasserstoff, Alkyl mit 1-3 Kohlenstoffatomen sowie Phenyl bedeutet.

$Z^1$ und $Z^2$ gleich oder verschieden sein können und für Wasserstoff, Alkyl mit 1-3 Kohlenstoffatomen, gegebenenfalls substitiertes Phenyl, Pyridyl, Alkoxycarbonyl mit 1-4 Kohlenstoffatomen im Alkohlteil, Cyano, Isocyano, Nitro, Formyl, ferner für Reste $COR^7$, $SOR^8$, $SO_2R^9$, $SO_2OR^{10}$ und $SO_2NR^{11}R^{12}$ stehen können

mit der Maßgabe, daß $Z^1$ und $Z^2$ nicht gleichzeitig Wasserstoff und Alkyl sind. Außerdem können $Z^1$ und $Z^2$ mit dem Kohlenstoffatom, mit dem sie verknüpft sind, einen 5-oder 6-gliedrigen Ring bilden, der als Ringglieder die Gruppen
-O-, -$\underset{O}{\underset{\|}{C}}$-, -$\underset{O}{\underset{\|}{S}}$-, -$SO_2$-, $>NR^{13}$ enthalten kann und an den Kohlenstoffatomen ein- oder zweifach durch $C_1$-$C_3$-Alkyl, Phenyl, Halogen, Nitro, Cyano oder Alkoxycarbonyl mit 1-3 Kohlenstoffatomen im Alkoholteil substituiert ist, wobei
$R^7$, $R^8$, $R^9$ und $R^{10}$ für Alkyl mit 1-3 Kohlenstoffatomen oder gegebenenfalls substituiertes Phenyl stehen und
$R^{11}$ und $R^{12}$ gleich oder verschieden sein können und für Wasserstoff, Alkyl mit 1-3 Kohlenstoffatomen oder gegebenenfalls substituiertes Phenyl stehen und
$R^{13}$ für $C_1$-$C_3$-Aklyl oder Phenyl steht,
dadurch gekennzeichnet, daß man Verbindungen der Formel II

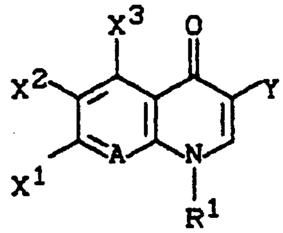

II

in der Y, A, $X^2$ und $X^3$ die oben angegebene Bedeutung haben und $X^1$ für Halogen, bevorzugt für Chlor und Fluor steht, mit Verbindungen der Formel III

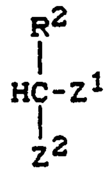

III

in der
$R^2$, $Z^1$ und $Z^2$ die oben angegebene Bedeutung haben in Gegenwart einer Base umsetzt.

4. Verfahren nach Anspruch 3, dadurch gekennzeichnet, daß als Basen Natriumamid, Natriumhydrid, Kalium-t.-butylat, n-Butyllithium oder Phenyllithium verwendet.

5. Verfahren nach den Ansprüchen 3 und 4, dadurch gekennzeichnet, daß man die Umsetzung bei 20 bis 200 °C durchführt.

6. Verfahren nach den Ansprüchen 3, 4, und 5, dadurch gekennzeichnet, daß man auf 1 Mol Carbonsäurederivat der Formel II 1 bis 5 Mol der Verbindung der Formel III einsetzt.

7. Verwendung von Chinoloncarbonsäuren und 1,8-Naphthyridin-4-on-carbonsäuren der Formel I gemäß Anspruch 1 zur Herstellung von Arzneimitteln.